# EUROPÄISCHE PATENTSCHRIFT

(11) **EP 0 680 946 B1**
(45) Veröffentlichungstag und Bekanntmachung des Hinweises auf die Patenterteilung: **16.12.1998**
(21) Anmeldenummer: 95106336.1
(22) Anmeldetag: 27.04.1995
(51) Int. Cl.: C07C 233/17, C07H 15/12, B01F 17/22

(54) **Salze von Alkyl- und Alkenylbernsteinsäurehalbamiden**
Alkyl- and alkenylsuccinic acid monoamide salts
Sels de monoamides de l'acide alkyl- et alkénylsuccinique

(30) Priorität: 04.05.1994 DE 4415703
(43) Veröffentlichungstag der Anmeldung: 08.11.1995
(73) Patentinhaber: Clariant GmbH, 65929 Frankfurt am Main (DE)
(72) Erfinder: Dönges, Reinhard, Dr., D-65812 Bad Soden (DE); Ehrler, Rudolf, Dr., D-65439 Flörsheim (DE); Milewski, Eckhard, D-65830 Kriftel (DE); Reng, Alwin K., D-65779 Kelkheim (DE)

(56) Entgegenhaltungen:
- WO-A-92/15554
- JP-A- 5 125 014

## Beschreibung

Die Erfindung betrifft Salze von Alkyl- und/oder Alkenylbernsteinsäurehalbamiden, ein Verfahren zu ihrer Herstellung sowie ihre Verwendung als Tenside.

Alkyl- und Alkenylbernsteinsäurehalbamide sind an sich seit langem bekannt. In der EP-A-0 498 178 sind Ammoniumsalze von Alkenylbernsteinsäurehalbamiden beschrieben, die als Korrosionsinhibitoren bzw. in der Öl- und/oder Gasförderung Verwendung finden.

Die Verwendung von Alkenylbernsteinsäurehalbamiden als Korrosionsschutzmittel ist ebenfalls in der EP-A-0 127 132 und in der EP-A-0 191 952 beschrieben.

In der EP-A-0 510 564 findet sich ein Verfahren zur Herstellung von oberflächenaktiven Mitteln aus Di- oder Tricarbonsäuren. Als Dicarbonsäure wird beispielsweise Bernsteinsäure verwendet. Die Produkte werden als oberflächenaktive Mittel, bevorzugt in kosmetischen Zubereitungen verwendet.

In der US-A-3 576 743 werden öllösliche Produkte beschrieben, die aus der Veresterung eines Polycarbonsäureanhydrids, insbesondere Alkenylbernsteinsäureanhydrid, mit einem mehrwertigen Alkohol und anschließender Umsetzung des gebildeten Esters mit einen primären Amin entstehen. Bei den eingesetzten Aminen handelt es sich um primäre Aminoalkohole, wie Ethanolamin, Tris(hydroxymethyl)aminomethan und Glucamin. Diese Produkte werden als Schmiermittel- und Kraftstoffadditive verwendet.

Die vorgenannten Verbindungen zeigen jedoch den Nachteil, nur in bestimmten Einsatzgebieten anwendbar zu sein. Oft weisen sie auch keine guten dermatologischen Eigenschaften auf, so daß sie für eine Anwendung als Tenside im Waschmittel- und Kosmetikbereich nicht in Frage kommen.

Es war daher Aufgabe der Erfindung, neue wasserlösliche Alkyl- und/oder Alkenylbernsteinsäureverbindungen zur Verfügung zu stellen, die die oben genannten Nachteile nicht aufweisen und die zudem biologisch gut abbaubar sind.

Aus JP-A-5125014 ist die Herstellung von 1-Octenylbernsteinsäure-N-methylglucamid und dessen Verwendung als oberflächenaktive Verbindung bekannt.

Gegenstand der Erfindung sind Salze von Alkyl- und/oder Alkenylbernsteinsäurehalbamiden der Formeln 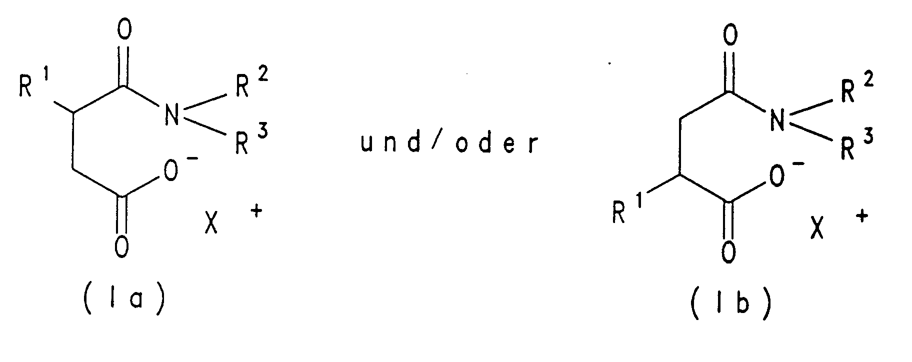 wobei
- R¹: C₆-C₁₀₀-Alkyl, C₆-C₁₀₀-Alkenyl,
- R²: einen Deoxysaccharidrest, der sich von Di- oder Oligosacchariden ableitet, bedeutet,
- R³: Wasserstoff, C₁-C₆-Alkyl oder R² bedeutet,
- X⁺: ein Proton, ein Alkalimetallion, das Äquivalent eines Erdalkalimetallions, ein Ammoniumion der Formel N⁺HR⁴R⁵R⁶ (II) oder der Formel N⁺HR²R³R⁴ (III) bedeutet,
wobei
R⁴, R⁵, R⁶ unabhängig voneinander für Wasserstoff, C₁-C₂₀-Alkyl, eine Hydroxygruppe, eine C₁-C₆-Hydroxyalkylgruppe oder C₃-C₆-Polyhydroxyalkylgruppe stehen und
R², R³, R⁴ die vorgenannten Bedeutungen haben.

Als Deoxysaccharid (auch Desoxysaccarid) wird nach der Nomenklatur in der organischen Chemie ein Saccharid bezeichnet, bzw. dem formal ein Hydroxylradikal durch ein Wasserstoffradikal ersetzt ist (D. Hellwinkel, "Die systematische Nomenklatur der Organischen Chemie", Springer Verlag, Berlin, Heidelberg, New York 1974, S. 70). Eine genauere Bezeichnung ist Deoxy-n-Saccharylrest, wobei n die Position innerhalb des Saccharylrestes kennzeichnet, die den Saccharidteil mit beispielsweise einem Amidstickstoff verknüpft.

Bevorzugt sind Salze von Bernsteinsäurehalbamiden der Formeln (Ia) und/oder (Ib) in denen
- R¹: C₆-C₂₂-Alkenyl,
- R²: einen Deoxysaccharidrest, der sich von einem, Stärkehydrolysat ableitet, bedeutet,
- R³: Wasserstoff oder C₁-C₆-Alkyl bedeutet oder = R² ist,
- X ⁺: ein Lithiumion, Kaliumion, Natriumion oder das Äquivalent eines Calcium- oder Magnesiumions bedeutet.

Ebenfalls Gegenstand der Erfindung ist ein Verfahren zur Herstellung von Salzen von Alkyl- und/oder Alkenylbernsteinsäurehalbamiden, der Formel Ia und/oder Ib, wobei R¹, R², R³ und X die zuvor angegebenen Bedeutungen haben und R³ zusätzlich einen von einem Mono-saccharid abgeleiteten Deoxysaccharidrest bedeutet, dadurch gekennzeichnet, daß man Alkyl- oder Alkenylbernsteinsäureanhydride der Formeln 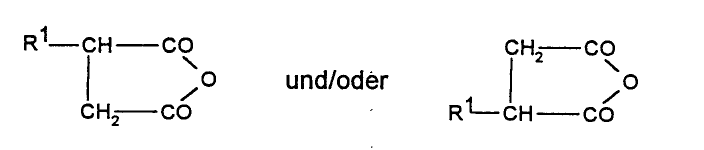 mit aminogruppenhaltigen Kohlehydraten der Formel

NHR²R³

in Wasser oder in wäßrig-alkoholischer Lösung und in Gegenwart von basischen Verbindungen umsetzt.

Bevorzugt werden als basische Verbindungen Alkalimetallhydroxide, Erdalkalimetallhydroxide, Alkylamine und/oder Hydroxyalkylamine verwendet. Besonders geeignet ist Natriumhydroxid. Weitere geeignete basische Verbindungen sind Amine, insbesondere Alkylamine, wie primäre, sekundäre und tertiäre C₁-C₆-Alkylamine, z.B. Triethylamin und Hydroxyalkylamine, wie primäre, sekundäre und tertiäre C₁-C₆-Hydroxyalkylamine, z.B. Triethanolamin sowie Aminozucker.

Die als Ausgangssubstanzen verwendeten Alkenylbernsteinsäureanhydride lassen sich durch Umsetzung von Maleinsäureanhydrid und Olefinen im Sinne einer En-Reaktion erhalten. Durch Hydrierung erhält man die entsprechenden Alkylbernsteinsäureanhydride.

Als aminogruppenhaltige Kohlenhydrate, die für die Umsetzung mit Bernsteinsäureanhydrid zu den gewünschten erfindungsgemäßen Verbindungen der Formeln (Ia) bzw. (Ib) geeignet sind, sind die Aminozucker und Aminopolyole, wobei im Sinne der Erfindung der Zuckerrest und der Polyolrest als Deoxysaccharidrest bezeichnet werden, zu nennen. Aminozucker, wie Glucosamin, Galaktosamin und andere, sind aus dem Stand der Technik bekannt und auf verschiedene Weise erhältlich (S. Peat, Adv. Carbohydr. Chem. 2, 37 (1946); Foster, Stacy, Adv. Carbohydr. Chem. 7, 247 (1952); Org. Synthesis 26, 36 (1946)). Aminopolyole sind ebenfalls seit langem bekannt und lassen sich durch reduktive Aminierung der entsprechenden Mono-, Di- oder Oligosaccharide erhalten (US-A-2 830 983, EP-A-0 255 033, US-A-2 016 962), so beispielsweise 1-Methylamino-1-deoxysorbit (N-Methylglucamin) oder 1-Amino-1-deoxygalactit.

Als Lösemittel für die vorgenannte Reaktion sind Wasser, niedere Alkohole, wie Methanol, Ethanol, Propanole, Glykole, weitere mit Wasser mischbare polare organische Lösemittel, wie Ketone und Glycolether, sowie als aprotische Lösemittel Dimethylformamid und Dimethylsulfoxid geeignet. Bevorzugt wird in einer wäßrigen Lösung oder einer wäßrig-alkoholischen Lösung umgesetzt. Die Umsetzung der Alkyl- und/oder Alkenylbernsteinsäureanhydride mit dem aminogruppenhaltigen Kohlenhydrat erfolgt bei einer Temperatur von 0 bis 100°C, vorzugsweise 30 bis 70°C.

Die Konzentration des aminogruppenhaltigen Kohlenhydrats in der Lösung beträgt 5 bis 70 Gew.-%, vorzugsweise 10 bis 50 Gew.-%.

Die aminogruppenhaltigen Kohlenhydrate werden in entsprechender Konzentration in einem der oben genannten Lösemittel vorgelegt und dazu dann das entsprechende Alkyl- und/oder Alkenylbernsteinsäureanhydrid zugetropft. Die Zutropfdauer beträgt zwischen 10 Minuten und 6 Stunden, bevorzugt zwischen 30 Minuten und 2 Stunden. Der pH-Wert der Reaktion wird über die Zugabe der basischen Verbindungen gesteuert.

Das Molverhältnis von Alkyl- und/oder Alkenylbernsteinsäureanhydrid zu aminogruppenhaltigem Kohlehydrat beträgt 0,8-1,2 : 0,8-1,2, bevorzugt jedoch 1:1.

Die erfindungsgemäßen Salze der Alkyl- und/oder Alkenylbernsteinsäurehalbamide finden Verwendung als Tenside. Die Salze der Alkyl- und/oder Alkenylbernsteinsäurehalbamide werden in einer Menge von 1 bis 25 Gew.-%, vorzugsweise 3 bis 15 Gew.-%, bezogen auf das Gesamtgewicht der Tensidlösung, verwendet.

### Analytik

Durch 12-stündiges Trocknen bei 100°C im Vakuumtrockenschrank wird der gesamte Feststoffgehalt der Lösung ermittelt.

Die Infrarot-Spektren der getrockneten Proben zeigen die typischen Banden der Amide bei ca. 1630 bis 1650 cm⁻¹ (sekundäre und tertiäre Amide) und zusätzlich bei ca. 1550 cm⁻¹ für das Carboxylat-Anion.

Die Analytik der Alkyl- und/oder Alkenylbernsteinsäurehalbamide ist in Beispiel 1 für die isolierten Trockensubstanzen wiedergegeben. Für die Beispiele 2 bis 11 wird der Feststoffgehalt wie oben beschrieben ermittelt. Daraus läßt sich der Halbamidgehalt mittels potentiometrischer Titration bestimmen.

### Beispiele

In den folgenden Beispielen wird die erfindungsgemäße Herstellung der durch die Formeln Ia und Ib beschriebenen Alk(en)ylbernsteinsäurederivate beschrieben. Bei den beschriebenen Verfahren entstehen grundsätzlich Produktgemische aus Ia und Ib, wobei Ia und Ib jeweils aufgrund zwei möglicher stereoisomerer Anordnungen des Restes R¹ (R und S) ein Diasteromerenpaar bilden. In Beispiel 1 werden die daraus resultierenden Isomeren einzeln bezeichnet, in den weiteren Beispielen wird nur die allgemeinere Bezeichnung wiedergegeben.

### Beispiel 1

Natriumsalz eines Gemisches aus
N-(1-Deoxyglucityl)-N-methyl-1(R)-dodecenylbernsteinsäuremonoamid
N-(1-Deoxyglucityl)-N-methyl-1(S)-dodecenylbernsteinsäuremonoamid
N-(1-Deoxyglucityl)-N-methyl-2(R)-dodecenylbernsteinsäuremonoamid
N-(1-Deoxyglucityl)-N-methyl-2(S)-dodecenylbernsteinsäuremonoamid

Es werden 97,5 g N-Methylglucamin in 790 ml Wasser vorgelegt. Dazu werden bei 60°C unter Rühren 133 g destilliertes Dodecenylbernsteinsäureanhydrid innerhalb von 4 Stunden zugetropft. Der pH wird mittels 40,3 g 50 Gew.-%iger Natronlauge zwischen 9,0 und 9,5 gehalten. Danach wird noch 1 Stunde bei 60°C nachgerührt. Man erhält eine stark schäumende, klare Lösung mit einem Feststoffgehalt von 25,4 Gew.-%. Eine getrocknete Probe zeigt im Infrarot-Spektrum die für die Formel Ia und Ib typischen Absorptionssignale bei 1412, 1575 und 1630 cm⁻¹.
10 g der obigen Lösung (entspricht 2,54 g Trockensubstanz) werden an einer Kieselgelsäule (Länge 50 cm, ID 5 cm) chromatographiert. Die Hauptfraktion (2,10 g) besteht nach HPLC aus den erwarteten 4 Komponenten im Verhältnis (in Flächen %) 19:17:33:31. Das Isomeren-Gemisch zeigt im ¹³C-NMR-Spektrum (75,4 MHz, CD₃OD) die erwarteten Signalgruppen für die Alkyl-Kette und C₂ und C₃ des Bernsteinsäureteils bei 14 bis 50 ppm, des Kohlenhydratteils von 64 bis 74 ppm, der olefinischen C-Atome bei 128 bis 135 ppm und der Carbonyl-C-Atome der Carboxy- und Amid-Gruppen bei 176 bis 185 ppm.

### Beispiel 2

### N-(1-Deoxyglucityl)-N-methyl-pentapropenylbernsteinsäuremonoamid Na-Salz

274 g Pentapropenylbernsteinsäureanhydrid (technisch, M = 366) werden mit 136 g N-Methylglucamin (1-Methylamino-1-deoxyglucit) in 105 g Wasser bei 60°C innerhalb 1 Stunde umgesetzt und 1 weitere Stunde nachgerührt. Danach wird innerhalb 1 Stunde 56 g 50 %ige Natronlauge zugegeben. Man erhält 583 g Lösung mit einem Feststoffgehalt von 68 Gew.-%. Der Halbamidgehalt des Feststoffs beträgt 78 Gew.-%.

### Beispiel 3

### N-(1-Deoxyglucityl)-N-methyl-tetrapropenylbernsteinsäuremonoamid Triethanolammoniumsalz

296 g Tetrapropenylbernsteinsäureanhydrid (technisch) der Molmasse 296, 196 g N-Methylglucamin (1-Methylamino-1-deoxyglucit) und 149 g Triethylamin werden bei 60°C in 200 g Wasser umgesetzt. Die Zutropfdauer des Bernsteinsäureanhydrids beträgt 1,5 Stunden. Man erhält eine Lösung mit 76 Gew.-% Feststoffgehalt. Der Halbamidgehalt des Feststoffs beträgt 82 Gew.-%.

### Beispiel 4

### N-(2-Deoxyglucosamino)-dodecyl/tetradecenylbernsteinsäuremonoamid Na-Salz

Es werden 64,7 g Glucosamin-Hydrochlorid in 300 ml Wasser vorgelegt. Dazu werden bei 10°C 88,8 g eines Gemisches aus Dodecenyl- und Tetradecenylbernsteinsäureanhydrides der mittleren Molmasse 296 innerhalb 1 Stunde zugetropft. Parallel dazu tropft man eine Lösung von 24 g Natriumhydroxid gelöst in 56 g Wasser so zu, daß ein pH-Wert von 7 bis 8 vorliegt. Man rührt noch eine Stunde bei 40°C nach und erhält 523 g einer klaren, stark schäumenden Lösung mit einem Feststoffgehalt von 32 Gew.-%. Der Halbamidgehalt des Feststoffs beträgt 88 Gew.-%.

### Beispiel 5

### N-(1-Deoxyglucityl)-N-methyl-dodecyl/tetradecenylbernsteinsäuremonoamid 1-Deoxyglucityl-dimethylammoniumsalz

Es werden 97,5 g N-Methylglucamin und 106 g Dimethylglucamin in 500 ml Wasser vorgelegt. Man heizt auf 60°C auf und tropft 148 g eines Gemisches aus Dodecenyl- und Tetradecenylbernsteinsäureanhydrides der mittleren Molmasse 296 innerhalb 1,5 Stunden zu. Man erhält 850 g einer klaren Lösung mit einem Feststoffgehalt von 43 Gew.-%. Der Halbamidgehalt des Feststoffs beträgt 78 Gew.-%.

### Beispiel 6

### N-(1-Deoxyglucityl)-N-methyl-dodecyl/tetradecenylbernsteinsäuremonoamid Triethanolammoniumsalz

148 g Dodecenyl/tetradecenylbernsteinsäureanhydrid (technisch) der Molmasse 296, 97,5 g N-Methylglucamin (1-Methylamino-1-deoxyglucit) und 74,6 g Triethanolamin werden bei 60°C in 500 ml Wasser umgesetzt. Die Zutropfdauer des Bernsteinsäureanhydrids beträgt zwei Stunden. Es resultieren 815 g Lösung mit 40 Gew.-% Feststoffgehalt. Der Halbamidgehalt des Feststoffs beträgt 77 Gew.-%.

### Beispiel 7

### N-(1-Deoxyglucityl)-N-methyl-dodecyl/tetradecenylbernsteinsäuremonoamid 1-Deoxyglucitylmethylammoniumsalz

Es werden 195 g N-Methylglucamin in 500 ml Wasser vorgelegt. Man heizt auf 60°C auf und tropft innerhalb von 1 Stunde 148 g eines Gemisches aus Dodecenyl- und Tetradecenylbernsteinsäureanhydrides der mittleren Molmasse 296 zu. Man rührt noch 1,5 Stunden bei 60°C nach und erhält 825 g einer klaren Lösung mit einem Feststoffgehalt von 41 Gew.-%. Der Halbamidgehalt des Feststoffs beträgt 88 Gew.-%.

### Beispiel 8

### N-(1-Deoxyglucityl)-N-methyl-dodecyl/tetradecenylbernsteinsäuremonoamid Na-Salz

Es werden 97,5 g N-Methylglucamin in 450 g Wasser vorgelegt. Dazu werden bei 60°C innerhalb 2 Stunden 148 g eines Gemisches aus Dodecenyl- und Tetradecenylbernsteinsäureanhydrides der mittleren Molmasse 296 zugetropft. Parallel dazu tropft man 61 g 30 %ige Natriumhydroxid-Lösung so zu, daß ein pH-Wert von 9 bis 10 vorliegt. Man rührt noch eine Stunde bei 60°C nach und erhält 743 g einer klaren, stark schäumenden Lösung mit einem Feststoffgehalt von 34 Gew.-%. Der Halbamidgehalt des Feststoffs beträgt 62 Gew.-%.

### Beispiel 9

### N-(1-Deoxyglucityl)-dodecyl/tetradecenylbernsteinsäuremonoamid Na-Salz

Es werden 90,5 g Glucamin in 450 g Wasser vorgelegt. Dazu werden bei 60°C innerhalb von 2 Stunden 148 g eines Gemisches aus Dodecenyl- und Tetradecenylbernsteinsäureanhydrides der mittleren Molmasse 296 zugetropft. Parallel dazu tropft man 70 g 30 %ige Natriumhydroxid-Lösung so zu, daß ein pH-Wert von 9 bis 10 vorliegt. Man rührt noch zwei Stunden bei 60°C nach und erhält 743 g einer klaren Lösung mit einem Feststoffgehalt von 31 Gew.-%. Der Halbamidgehalt des Feststoffs beträgt 92 Gew.-%.

### Beispiel 10

### N-(2-Deoxy-D-glucopyranosylmannityl/sorbityl)dodecenylbernsteinsäuremonoamid Na-Salz

Es werden 34,1 g 2-Amino-2-deoxy-D-glucopyranosylmannit/sorbit (2-Aminopalatinit) in 87 g Wasser vorgelegt. Dazu werden bei 50°C 23,1 g eines Dodecenylbernsteinsäureanhydrid innerhalb von ½ Stunde zugetropft. Parallel dazu tropft man 7,5 g 50 %ige Natriumhydroxid-Lösung so zu, daß ein pH-Wert von 9 bis 9,5 gehalten wird. Man rührt noch zwei Stunden bei 50°C nach und erhält 140,5 g einer klaren, stark schäumenden Lösung mit einem Feststoffgehalt von 46 Gew.-%. Der Halbamidgehalt des Feststoffs beträgt 88 Gew.-%.

### Beispiel 11

### N-(1-Deoxyglucityl)-N-methyl-dodecyl/tetradecenylbernsteinsäuremonoamid Trishydroxymethyl-methylammoniumsalz

195 g N-Methylglucamin werden in 800 ml Wasser suspendiert. Unter Eiskühlung läßt man langsam 296 g eines Gemisches aus Dodecyl- und Tetradecenylbernsteinsäureanhydrides (mittlere Molmasse 296) zutropfen (Dauer: 3 Stunden) und hält dabei mit 80 g 50 %iger Natronlauge den pH-Wert von 9 bis 10. Es wird noch eine Stunde bei 60°C gerührt. Die nun klare Lösung wird unter Eiskühlung mit 50 g konzentrierter Schwefelsäure auf pH 3 angesäuert und vom ausgefallenen Natriumsulfat abfiltriert. Das Filtrat wird mit 112 g Trishydroxymethylaminomethan neutralisiert (pH-Wert 6,6). Das Methanol wird abdestilliert und dabei sukzessiv durch Wasser ersetzt. Man erhält eine Lösung mit einem Feststoffgehalt von 39 Gew.-%. Der Halbamidgehalt des Feststoffs beträgt 91 Gew.-%.

### Beispiele 12 bis 16

### Prüfung des Schaumvermögens und Bestimmung des Kältetrübungspunktes und des Zeïn-Wertes der Beispiele 5 bis 9.

Die Zeïn-Werte (A. K. Reng et al., Parfümerie und Kosmetik 68, 771 bis 788, 1987) geben orientierende Hinweise über die Hautverträglichkeit von Tensiden.

Für milde Tenside sollten sie kleiner als 200 mg Stickstoff/100 ml Tensidlösung betragen. Die erfindungsgemäß hergestellten Produkte der Beispiele 5 bis 9 weisen Zeïnwerte weit unter dieser Grenze auf und sind somit als sehr mild einzustufen. Die Prüfungsergebnisse sind in Tabelle 1 zusammengefaßt.

**Tabelle 1**

| Beispiel | Produkt aus Beispiel | Schaum^{a)} sofort / 5 min. | Kältetrübungspunkt | Zeïn-Wert |
|---|---|---|---|---|
| 12 | 5 | 230 / 230 | -5°C | 32 |
| 13 | 6 | 240 / 240 | 2°C | 46 |
| 14 | 7 | 260 / 250 | -3°C | 26 |
| 15 | 8 | 250 / 250 | -4°C | 49 |
| 16 | 9 | 250 / 250 | -8°C | 123 |

| | | | | |
|---|---|---|---|---|
| a) nach Ross/Miles in mm bei 1,0 % waschaktiver Substanz. | | | | |

### Beispiele 17a und b

Herstellung besonders hautmilder Haarshampoos unter Verwendung der Produkte aus Beispiel 7 und Beispiel 9 (alle Angaben in Gew.-%).

| Beispiel 17a | |
|---|---|
| ®Genapol LRO flüssig (Laurylalkoholethersulfat, Hersteller: Hoechst AG) | 40 % |
| Parfümöl | 0,03 % |
| Wasser | 47,65 % |
| Produkt aus Beispiel 7 (als 41 gew.-%ige Lösung) | 8,5 % |
| ®Genapol L-3 (Laurylalkoholethoxylat, Hersteller: Hoechst AG) | 2.0 % |
| Farbstoff-Lösung | 0,27 % |
| Konservierungsmittel | 0,05 % |
| Natriumchlorid | 1,5 % |

| Beispiel 17b | |
|---|---|
| ®Genapol LRO flüssig (Laurylalkoholethersulfat) | 40 % |
| Parfümöl | 0,03 % |
| Wasser | 44,85 % |
| Produkt aus Beispiel 9 (als 31 gew.-%ige Lösung) | 11,3 % |
| ®Genapol L-3 (Laurylalkoholoxethylat) | 2.0 % |
| Farbstoff-Lösung | 0,27 % |
| Konservierungsmittel | 0,05 % |
| Natriumchlorid | 1,5 % |

### Beispiele 18a bis d

Herstellung besonders hautmilder Spülmittelkonzentrate unter Verwendung des Produktes aus Beispiel 7.

| Beispiel | 18a | 18b | 18c | 18d |
|---|---|---|---|---|
| | Anteile in Gew.-% | | | |
| Sek. Alkansulfonat (®Hostapur SAS, 60 % Hersteller: Hoechst AG) | 45,0 | 33,3 | - | - |
| Laurylethersulfat (®Genapol LRO, 70 % Hersteller: Hoechst AG) | 11,4 | - | 38,6 | 14,2 |
| Fettalkoholoxethylat (®Genapol UD 80 Hersteller: Hoechst AG) | - | 10,0 | - | 7,0 |
| Cocosamidopropylbetain (®Genagen CAB, 30 % Hersteller: Hoechst AG) | - | - | 10,0 | 10,0 |
| Produkt-Beispiel 7 (als 41 gew.-%ige Lösung) | 12,2 | 24,4 | 24,4 | 48,8 |
| Ethanol | 5,0 | 5,0 | 5,0 | 5,0 |
| Wasser | 26,4 | 27,3 | 22,0 | 15,0 |

## Patentansprüche

1. Salze von Alkyl- und/oder Alkenylbernsteinsäurehalbamiden der Formeln 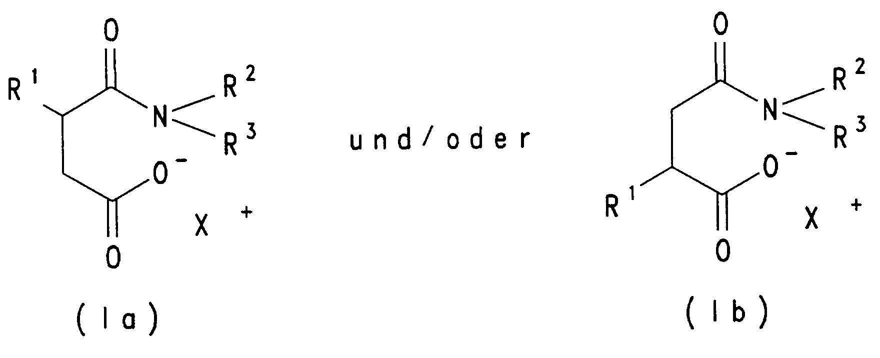 wobei
R¹ C₆-C₁₀₀-Alkyl, C₆-C₁₀₀-Alkenyl (geradkettig oder verzweigt),
R² einen Deoxysaccharidrest, der sich von Di- oder Oligosacchariden ableitet, bedeutet,
R³ Wasserstoff oder C₁-C₆-Alkyl oder R² bedeutet,
X⁺ ein Proton, ein Alkalimetallion, ein Äquivalent eines Erdalkalimetallions, ein Ammoniumion der Formel N⁺HR⁴R⁵R⁶ (II) oder der Formel N⁺HR²R³R⁴ (III) bedeutet,
wobei
R⁴, R⁵, R⁶ unabhängig voneinander für Wasserstoff, C₁-C₂₀-Alkyl, eine Hydroxygruppe, C₁-C₆-Hydroxyalkyl oder C₃-C₆-Polyhydroxyalkyl stehen, und
R², R³, R⁴ die vorgenannte Bedeutung haben.

2. Salze von Bernsteinsäurehalbamiden nach Anspruch 1, wobei
R¹ C₆-C₂₂-Alkenyl,
R² einen Deoxysaccharidrest, der sich von einem Stärkehydrolysat ableitet, bedeutet,
R³ Wasserstoff, C₁-C₆-Alkyl bedeutet oder = R² ist,
X⁺ ein Lithiumion, Kaliumion, Natriumion oder das Äquivalent eines Calcium- oder Magnesiumions bedeutet.

3. Verfahren zur Herstellung von Salzen von Alkyl- und/oder Alkenylbernsteinsäurehalbamiden, der Formel Ia und/oder Ib gemäß Anspruch 1, wobei R¹, R², R³ und X die in Anspruch 1 angegebenen Bedeutungen haben und R³ zusätzlich einen von einem Mono-saccharid abgeleiteten Deoxysaccharidrest bedeutet, dadurch gekennzeichnet, daß man Alkyl- oder Alkenylbernsteinsäureanhydride der Formeln 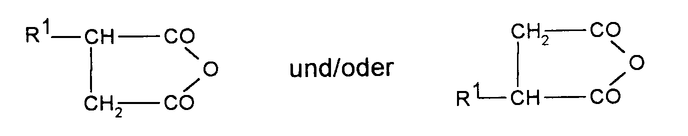 mit aminogruppenhaltigen Kohlehydraten der Formel
NHR²R³
in Wasser oder in wäßrig-alkoholischer Lösung und in Gegenwart von basischen Verbindungen umsetzt.

4. Verfahren nach Anspruch 3, dadurch gekennzeichnet, daß als basische Verbindungen Alkalimetallhydroxide, Erdalkalimetallhydroxide, Alkylamine und/oder Hydroxyalkylamine verwendet werden.

5. Verfahren nach Anspruch 3 oder 4, dadurch gekennzeichnet, daß man bei einer Temperatur von 0 bis 100 °C, vorzugsweise 30 bis 70 °C, umsetzt.

6. Verfahren nach einem der Ansprüche 3 bis 5, dadurch gekennzeichnet, daß die Konzentration des aminogruppenhaltigen Kohlenhydrats in der Lösung 5 bis 70 Gew.-%, vorzugsweise 10 bis 50 Gew.-% beträgt.

7. Verfahren nach einem der Ansprüche 3 bis 6, dadurch gekennzeichnet, daß das Molverhältnis von Alkyl- und/oder Alkenylbernsteinsäureanhydrid zu aminogruppenhaltigem Kohlenhydrat 0,8 - 1,2, : 0,8 - 1,2, vorzugsweise 1 : 1 beträgt.

8. Verwendung der Salze der Alkyl- und/oder Alkenylbernsteinsäurehalbamide der Ansprüche 1 bis 2 als Tenside.

9. Verwendung nach Anspruch 8, dadurch gekennzeichnet, daß die Salze der Alkyl- und/oder Alkenylbernsteinsäurehalbamide in einer Menge von 1 bis 25 Gew.-%, vorzugsweise 3 bis 15 Gew.-%, bezogen auf das Gesamtgewicht der Tensidlösung, verwendet werden.

## Claims

1. A salt of an alkyl- and/or alkenylsuccinic acid half-amide of the formula 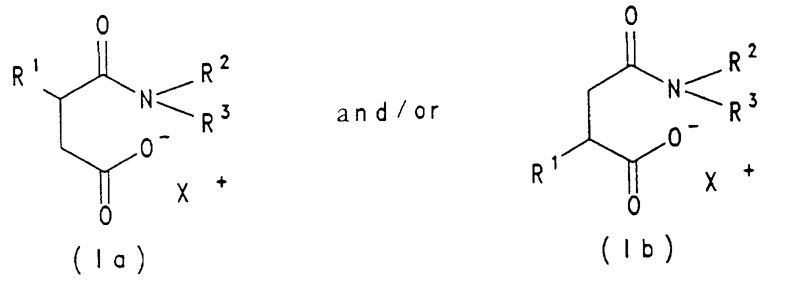 in which
R¹ is C₆-C₁₀₀-alkyl or C₆-C₁₀₀-alkenyl (straight-chain or branched),
R² is a deoxysaccharide radical which is derived from di- or oligosaccharides,
R³ is hydrogen, C₁-C₆-alkyl or R², and
X⁺ is a proton, an alkali metal ion, one equivalent of an alkaline earth metal ion, or an ammonium ion of the formula N⁺HR⁴R⁵R⁶ (II) or of the formula N⁺HR²R³R⁴ (III),
in which
R⁴, R⁵ and R⁶ independently of one another are hydrogen, C₁-C₂₀-alkyl, a hydroxyl group, C₁-C₆-hydroxyalkyl or C₃-C₆-polyhydroxyalkyl and
R², R³ and R⁴ have the abovementioned meanings.

2. A salt of a succinic acid half-amide as claimed in claim 1, in which
R¹ is C₆-C₂₂-alkenyl,
R² is a deoxysaccharide radical which is derived from starch hydrolysate,
R³ is hydrogen or C₁-C₆-alkyl, or equals R², and
X⁺ is a lithium ion, potassium ion or sodium ion or the equivalent of a calcium or magnesium ion.

3. A process for the preparation of a salt of an alkyl- and/or alkenylsuccinic acid half-amide, of the formula Ia and/or Ib as claimed in claim 1, in which R¹, R², R³ and X have the meanings given in claim 1 and R² is additionally a deoxysaccharide radical which is derived from a monosaccharide, which comprises reacting an alkyl- or alkenylsuccinic acid anhydride of the formula 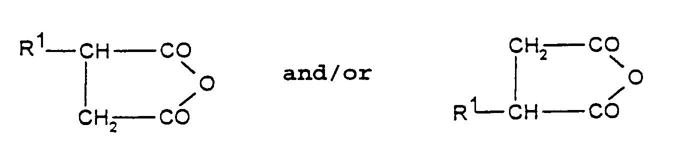 with a carbohydrate containing amino groups of the formula
NHR²R³
in water or in aqueous-alcoholic solution and in the presence of a basic compound.

4. The process as claimed in claim 3, wherein the basic compound is an alkali metal hydroxide, alkaline earth metal hydroxide, alkylamine or hydroxyalkylamine.

5. The process as claimed in claim 3 or 4, wherein the reaction is carried out at a temperature of 0 to 100°C, preferably 30 to 70°C.

6. The process as claimed in one of claims 3 to 5, wherein the concentration of the carbohydrate containing amino groups in the solution is 5 to 70% by weight, preferably 10 to 50% by weight.

7. The process as claimed in one of claims 3 to 6, wherein the molar ratio of alkyl- and/or alkenylsuccinic acid anhydride to carbohydrate containing amino groups is 0.8-1.2:0.8-1.2, preferably 1:1.

8. The use of a salt of an alkyl- and/or alkenylsuccinic acid half-amide as claimed in claims 1 to 2 as a surfactant.

9. The use as claimed in claim 8, where the salt of an alkyl- and/or alkenylsuccinic acid half-amide is used in an amount of 1 to 25% by weight, preferably 3 to 15% by weight, based on the total weight of the surfactant solution.

## Revendications

1. Sels d'hémi-amides de l'acide alkyl- et/ou alcénylsuccinique de formules 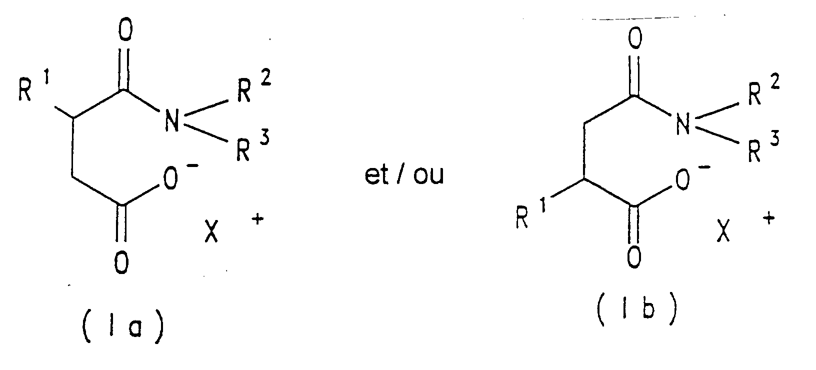 dans lesquelles
R¹ représente un groupe alkyle en C₆-C₁₀₀, alcényle en C₆-C₁₀₀ (à chaîne linéaire ou ramifiée),
R² représente un groupe désoxysaccharide, qui dérive de di- ou d'oligosaccharides,
R³ représente un atome d'hydrogène ou un groupe alkyle en C₁-C₆ ou R²,
X⁺ représente un proton, un ion d'un métal alcalin, un équivalent d'un ion de métal alcalino-terreux, un ion ammonium de formule N⁺HR⁴R⁵R⁶ (II) ou de formule N⁺HR²R³R⁴ (III),
dans lesquelles
R⁴, R⁵, R⁶ représentent indépendamment l'un de l'autre un atome d'hydrogène, un groupe alkyle en C₁-C₂₀, un groupe hydroxy, hydroxyalkyle en C₁-C₆ ou polyhydroxyalkyle en C₃-C₆ et
R², R³, R⁴ ont la signification citée précédemment.

2. Sels d'hémi-amides de l'acide succinique selon la revendication 1, dans lesquels
R¹ représente un groupe alcényle en C₆-C₂₂,
R² représente un groupe désoxysaccharide, qui dérive d'un hydrolysat d'amidon,
R³ représente un atome d'hydrogène, un groupe alkyle en C₁-C₆ ou est = R²,
X⁺ représente un ion lithium, un ion potassium, un ion sodium ou l'équivalent d'un ion calcium ou magnésium.

3. Procédé de préparation de sels d'hémi-amides de l'acide alkyl- et/ou alcénylsuccinique, de formule Ia et/ou Ib selon la revendication 1, dans lequel R¹, R², R³ et X ont les significations données à la revendication 1 et R³ représente en plus un groupe désoxysaccharide dérivé d'un monosaccharide, caractérisé en ce que, l'on met à réagir un anhydride d'acide alkyl- et/ou alcénylsuccinique de formules 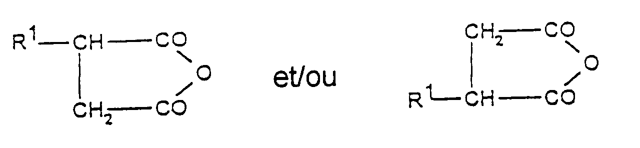 avec des hydrates de carbone contenant des groupes amino de formule
NHR²R³
dans l'eau ou dans une solution aqueuse alcoolique et en présence de composés basiques.

4. Procédé selon la revendication 3, caractérisé en ce qu'on utilise comme composés basiques des hydroxydes de métaux alcalins, des hydroxydes de métaux alcalino-terreux, des alkylamines et/ou des hydroxyalkylamines.

5. Procédé selon la revendication 3 ou 4, caractérisé en ce qu'on effectue la réaction à une température de 0 à 100°C, de préférence de 30 à 70°C.

6. Procédé selon l'une des revendications 3 à 5, caractérisé en ce que la concentration des hydrates de carbone contenant des groupes amino est de 5 à 70% en poids, de préférence de 10 à 50% en poids dans la solution.

7. Procédé selon l'une des revendications 3 à 6, caractérisé en ce que le rapport molaire du l'anhydride alkyl- et/ou alcénylsuccinique à l'hydrate de carbone contenant des groupes amino est de 0,8 - 1,2:0,8 - 1,2, de préférence de 1:1.

8. Utilisation des sels des hémi-amides de l'acide alkyl- et/ou alcénylsuccinique selon les revendications 1 à 2 comme agents tensioactifs.

9. Utilisation selon la revendication 8, caractérisée en ce qu'on utilise les sels des hémi-amides de l'acide alkyl- et/ou alcénylsuccinique en une quantité de 1 à 25% en poids, de préférence de 3 à 15% en poids, par rapport au poids total de la solution de tensioactif.
